# EUROPEAN PATENT SPECIFICATION

(11) **EP 3 709 956 B1**
(45) Date of publication and mention of the grant of the patent: **23.10.2024**
(21) Application number: 18800089.7
(22) Date of filing: 07.11.2018
(51) Int. Cl.: A61K 8/35, A61K 8/44, A61K 8/49, A61K 8/60, A61Q 17/04, A61Q 19/04, A61K 8/895

(54) **COSMETIC OR DERMATOLOGICAL COMPOSITIONS**
KOSMETISCHE ODER DERMATOLOGISCHE ZUSAMMENSETZUNGEN
COMPOSITIONS COSMÉTIQUES OU DERMATOLOGIQUES

(30) Priority: 13.11.2017 EP 17201411
(43) Date of publication of application: 23.09.2020
(73) Proprietor: DSM IP Assets B.V., 6221 BE Maastricht (NL)
(72) Inventor: IMFELD, Dominik, 4303 Kaiseraugst (CH); JACKSON, Eileen, 4303 Kaiseraugst (CH); LAURENT, Guillaume Bernard, 4303 Kaiseraugst (CH); SCHENK, Kerstin Katharina, 4303 Kaiseraugst (CH)
(74) Representative: dsm-firmenich IP
(86) International application number: PCT/EP2018/080383
(87) International publication number: WO 2019/091992

(56) References cited:
- WO-A1-2010/020626
- DE-A1- 102007 017 438
- DE-A1- 102007 031 661
- US-A1- 2005 100 516
- US-A1- 2011 097 288

## Description

The present invention relates to novel cosmetic or dermatological compositions comprising a polysiloxane based UV filter having a chromophore residue of the benzalmalonate type and least one self-tanning agent. The compositions are suitable for artificial/sunless tanning and/or browning of human skin as well as for restoring and/ or improving the skin barrier function.

By the term "self-tanning agent" or "artificial/sunless tanning agent" are intended agents which, when topically applied onto the skin, in particular onto the face, elicit a tanning effect with an appearance more or less similar to that resulting from prolonged exposure to the sun (natural tanning) or under a UV lamp.

It is today important to look well and a tanned skin is always a sign of good health. However, natural tanning is not always desirable insofar as it requires prolonged exposure to UV radiation which causes browning of the skin but, on the other hand, induces skin damages such as increased wrinkling, elastosis, pigmentary changes, precancerous and cancerous skin lesions. Thus, it is desirable to have an alternative to natural tanning. Therefore, self-tanning ingredients are gaining more importance for various applications in the skin and sun care market.

The majority of cosmetic products for artificial tanning of the skin (i.e. self-tanners) contain as active component (i.e. as self-tanning agent) carbonyl derivatives which permit the formation of colored compounds by interaction with the amino acids of the skin. These self-tanning agents include mono- or polycarbonyl compounds, such as, for example, isatin, alloxan, ninhydrin, glyceraldehyde, mesotartaric aldehyde, glutaraldehyde, erythrulose and dihydroxyacetone (DHA).

Currently available self-tanning ingredients such as in particular erythrulose or dihydroxyacetone, however, tend to deteriorate the barrier function of the skin leading to an increase water loss of the skin which can be measured by an increased transepidermal water loss (TEWL). High TEWL values, as a marker of disturbed skin barrier function, are furthermore frequently correlated with low hydration of the stratum corneum, i.e. a decreased moisturization of the skin.

WO2010/020626 discloses compositions comprising at least one specific titanium dioxide and at least one self-tanning agent and a cosmetically acceptable carrier. The compositions are in particular suitable for artificial/sunless tanning and/or browning of human skin.

Today's self-tanning ingredients furthermore tend to sink into the skin such as in particular into dry skin, leaving dark patches that look entirely unnatural.

Thus, there is an ongoing need for efficient self-tanners, which counteract the skin barrier deterioration caused by self-tanning agents and are thus able to restore respectively to improve the barrier function of the skin in order to maintain skin integrity, health and appearance. Furthermore, there is a need of ingredients which enhance the tanning efficacy of self-tanning agents allowing the reduction of their use levels to minimize potential adverse effects thereof.

It has now surprisingly been found, that the addition of a polysiloxane based UV-filter having a chromophore residue of the benzalmalonate type to cosmetic compositions comprising a self-tanning agent overcomes the shortcomings of the prior art by significantly enhancing the skin tanning efficacy of the self-tanning agent, while at the same time restoring the barrier function of the skin and are thus useful for improving skin integrity, health and appearance. Such compositions are accordingly particularly suitable for tanning of dry skin.

Thus, the invention relates to a cosmetic or dermatological composition comprising an effective amount of erythrulose and an effective amount of a polysiloxane based UV-filter having a chromophore residue of the benzalmalonate typeas defined in claim. Furthermore, the invention relates to the use of such compositions for artificial/sunless tanning and/ or browning of human skin as well as for restoring and/ or improving the skin barrier function.

It is well understood that in all embodiments of the present invention the polysiloxane based UV-filter having a chromophore residue of the benzalmalonate type is used a distinct, separate UV filter and not in the form of a coating on a titanium dioxide, which coating is adhered to the surface of the titanium dioxide.

The invention also relates to the topical application of compositions according to the invention for the coloring/browning of the skin to impart an appearance similar to natural tanning of the skin, for enhancement of the natural glow of the skin, for providing a healthy appearance of the skin and/ or for restoring and/ or improving the skin barrier function.

In all embodiments of the present invention preferably the compositions are free of (i.e. do not contain) a titanium dioxide coated with a polysiloxane based UV-filter having a chromophore residue of the benzalmalonate type such as in particular polysilicone-15, which combination leads to an unwanted coloration of the composition.

In all embodiments of the present invention, the compositions even more preferably do not contain a titanium dioxide which is substantially free of any aluminium coating. In an even more preferred embodiment, the compositions according to the present invention are completely free of (i.e. do not contain) any titanium dioxide (coated or uncoated) as the combination thereof leads to an unwanted coloration of the composition.

According to certain embodiments, restoring and/ or improving the skin barrier function comprises at least one of restoring, maintaining or reducing the skin dehydration rate and restoring, maintaining or promoting the skin hydration value.

According to other embodiments, the cosmetic or dermatological compositions according to the present invention are effective in at least one of improving the skin health, skin integrity, skin firmness, skin extensibility, skin elasticity, beautification of overall skin appearance and any combination thereof. Each possibility represents a separate embodiment of the present invention

The present invention also relates to a method for artificially tanning or browning of the skin as well as moisturizing the skin said method comprising the topical application of an effective amount of a composition according to the invention.

Furthermore, the invention relates to the use of a polysiloxane-based UV filter having a chromophore residue of the benzalmalonate type for restoring and/ or improving the barrier function of skin treated with a erythrulose such as in particular to restore respectively to reduce the skin dehydration rate and to restore respectively improve the skin hydration value, compared to the treatment with the self-tanning agent alone.

In another embodiment, the invention relates to the use of a polysiloxane-based UV filter having a chromophore residue of the benzalmalonate type for enhancing the tanning efficiency of a self-tanning agent such as in particular erythrulose and optionally appreciating the effect. The enhancement of the skin tan by the inventive compositions can be assessed by measuring the luminosity (L-value) of the skin with a chromameter.

In a further embodiment, the invention relates to the use of a polysiloxane-based UV filter having a chromophore residue of the benzalmalonate type such as in particular polysilicone-15 in combination with a self-tanning agent such as in particular erythrulose in a cosmetic or dermatological composition for (synergistically) reducing the luminosity (L-value) of the skin treated with the inventive composition and optionally appreciating the effect. In particular the luminosity is reduced by at least 0.5, more preferably by at least 1. The luminosity is measured after 24h using a chromameter and compared to the respective composition comprising only the benzalmalonate type such as in particular polysilicone-15 or the self-tanning agent such as in particular erythrulose.

The term 'effective amount' as used herein refers to an amount necessary to obtain a physiological effect. The physiological effect may be achieved by one single dose or by repeated doses. The dosage administered may, of course, vary depending upon known factors, such as the physiological characteristics of the particular composition and its mode and route of administration; the age, health and weight of the recipient; the nature and extent of the symptoms; the kind of concurrent treatment; the frequency of treatment; and the effect desired and can be adjusted by a person skilled in the art.

Preferred polysiloxane-based UV filters according to the present invention are linear or cyclic polysiloxane compounds according to formula Ia or Ib: 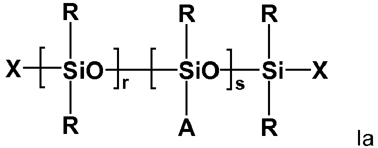 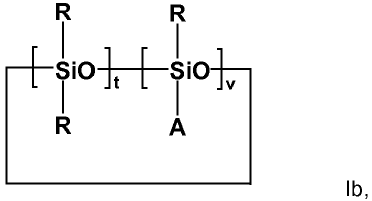 wherein
- X: is R or A;
- A: is selected from formula Ila and/ or IIb or IIe: 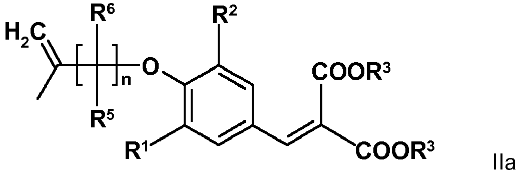 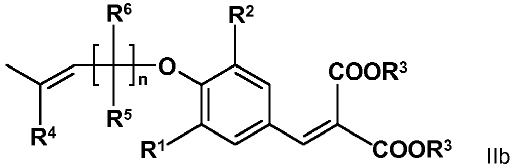 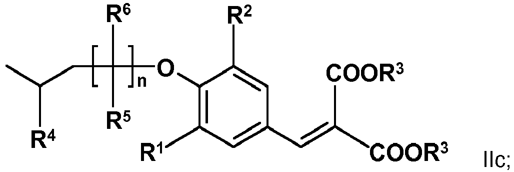
- R: is hydrogen, C₁₋₆-alkyl or phenyl;
- R¹ and R²: are each independently hydrogen, hydroxy, C₁₋₆-alkyl or C₁₋₆-alkoxy;
- R³: is C₁₋₆-alkyl;
- R⁴: is hydrogen or C₁₋₆-alkyl;
- R⁵ and R⁶: are each independently hydrogen or C₁₋₆-alkyl;
- r: is from 0 to 250;
- s: is from 0 to 20;
- r + s: is at least 3;
- t: is from 0 to 10;
- v: is from 0 to 10;
- v + t: is at least 3; and
- n: is from 1 to 6;
with the proviso that when s is 0, at least one X is A.

The term "C₁₋₆-alkyl" refers to groups such as methyl, ethyl, propyl, isopropyl, butyl, sec. butyl, isobutyl, pentyl and neopentyl. The term "C₁₋₆-alkoxy" refers to the corresponding alkoxy groups.

In all embodiments of the present invention, R is preferably methyl.

The residues R¹ and R² are preferably hydrogen, methoxy or ethoxy, more preferably hydrogen, or one of R¹ and R² is hydrogen and the other is methyl, methoxy or ethoxy.

The residues R³ are preferably methyl or ethyl, more preferably ethyl.

Preferably, R⁴ is hydrogen or methyl, R⁵ and R⁶ are hydrogen and n is 1.

The polysiloxane compounds having a group A of the general formula IIa and IIb and their preparation are described in European patent application EP-A0538431. These polysiloxane compounds are most preferred.

The polysiloxane compounds having a group A of the general formula IIc and their preparation are described in the European patent application EP-A 0358 584.

In the linear polysiloxane compounds according to formula Ia the chromophore carrying residue A may be connected to the end groups of the polysiloxane (X=A) or may be statistically distributed over the polymer.

Linear polysiloxane compounds wherein the chromophore carrying residue A is statistically distributed are preferred. Said preferred polysiloxane compounds have at least one unit carrying the chromophore residue (s = 1), preferably s has a value of from about 2 to about 10, more preferably a statistical mean of about 4. The number of r of the other silicone units present in the polysiloxane compounds is preferably about 5 to about 150, more preferably a statistical mean of about 60.

Polysiloxane compounds wherein 20% or less, preferably less than 10%, of the total siloxane units are units carrying a chromophore residue are preferred with respect to cosmetic properties.

The ratio of polysiloxane units having a chromophore residue A of the formula Ila to those having a chromophore residue A of the formula Iib is not critical. Said ratio may be about 1:1 to about 19:1, preferably about 2:1 to about 9:1, more preferably about 4:1.

The polysiloxane compounds Ia or Ib wherein A is a reside of the formula IIa or IIb can be prepared as described in EP-B0538431 by silylation of the corresponding benzalmalonates according to the following reaction scheme: 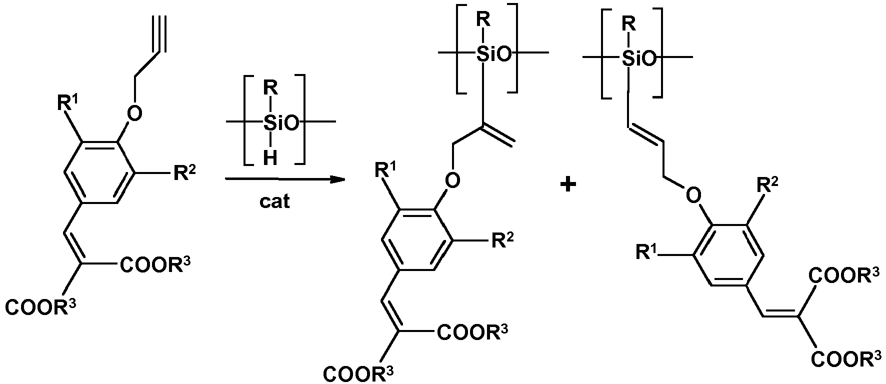 wherein R¹, R² and R³ are as defined above.

The silylation of the 4-(2-propinyloxy)phenyl methylene diethylester may be carried out employing known procedures for the addition of silicon bonded hydrogen atoms to groups containing aliphatic unsaturation. Such reactions are generally catalyzed by a platinum group metal or a complex of such a metal. Examples of catalysts which may be employed are platinum on carbon, chloroplatinic acid, platinum acetyl acetonate, complexes of platinum compounds with unsaturated compounds e.g. olefins and divinyl disiloxanes, complexes of rhodium and palladium compounds and complexes of platinum compounds supported on inorganic substrates. The addition reaction may be performed at reduced, atmospheric or increased pressure. A solvent can be used, e.g. toluene or xylene, in the reaction mixture although the presence of the solvent is not essential. It is also preferred to carry out the reaction at elevated reaction temperatures e.g. from about 50°C to about 150°C.

Particularly preferred are compounds of the general formula la, wherein
- X: signifies methyl,
- A: signifies the group of the formula Ila or Iib,
- R: signifies methyl,
- R¹ and R²: signify hydrogen, methoxy or ethoxy or one or R¹ and R² is hydrogen and the other is methyl, methoxy or ethoxy,
- R³: signifies methyl or ethyl,
- R⁴: signifies hydrogen or methyl,
- R⁵ and R⁶: signify hydrogen,
- r: is about 5 to 150,
- s: is about 2 to about 10, and
- n: has a value of 1.

Most preferred are linear polysiloxanes of the general formula la, wherein
- X: signifies methyl,
- A: signifies a group of the formula Ila or IIb,
- R: signifies methyl,
- R¹ and R²: signify hydrogen,
- R³: signifies ethyl,
- R⁴: signifies hydrogen,
- R⁵ and R⁶: signify hydrogen,
- r: is a statistical mean of about 60,
- s: is a statistical mean of about 4, and
- n: has a value of 1.

These most preferred polysiloxane-based UV filter in all embodiments of the present invention is polysilicone-15 (INCI) which is commercially available under the tradename PARSOL^{®} SLX at DSM Nutritional Products Ltd.

The self-tanning agent is generally selected from among mono- polycarbonyl one derivatives as described in Preferably, DHA and/ or erythrulose (in D- or L-form or as the racemate),particular erythrulose.

The self-tanning agent| can be used in combination with at least one synthetic or natural direct dye and/or at least one indole derivative, such as those described in EP-425,324 and EP-456,545.

These self-tanning agent| can also be used in combination with other synthetic or natural agents for coloring the skin.

By the term "agent for coloring the skin" is intended any compound having a specific affinity for the skin and which imparts thereto a lasting and noncovering (namely, having no tendency to opacify the skin) coloring, which is removed neither with water nor using a solvent, and which withstands both rubbing and washing with a solution comprising surfactants. Such a lasting coloring is therefore distinguished from the superficial and short-lived coloring contributed, for example, by a makeup pigment.

The additional coloring agents can also be selected, for example, from among plant extracts, such as, for example, extracts of "insoluble" redwoods of the Pterocarpus genus and of the Baphia genus, such as Pterocarpus santalinus, Pterocarpus osun, Pterocarpus soyauxii, Pterocarpus erinaceus, Pterocarpus indicus or Baphia nitida, such as those described in EP-971,683.

The coloring agents can also be iron oxide nanopigments for which the mean size of the individual particles is less than 100 nm, such as those described in EP-966,953.

The term erythrulose refers to erythrulose in D- or L-form or as the racemate. Preferably in all embodiments according to the present invention L-(+)-Erythrulose [533-50-6] is used. Erythrulose is e.g. commercially available as ERYTHRULOSE at DSM Nutritional Products Ltd, Kaiseraugst (at least 50% of Erythrulose in water). Even more preferably, in all embodiments of the present invention erythrulose is either used a sole self-tanning agent or in combination with DHA (i.e. as combination of erythrulose and DHA as sole self-tanning agent).

The amount of the polysiloxane-based UV filter having a chromophore residue of the benzalmalonate type such as preferably polysilicone-15 in the compositions according to the present invention is preferably selected in the range 0.1 to 15 wt.-%, such as in the range of 0.5 to 10 wt.-%, such as most preferably in the range of 1 to 5 wt.-% based on the total weight of the composition.

The amount of the at least one self-tanning agent in the compositions according to the present invention is preferably selected in the range of 0.1 to 20 wt.-%, preferably from 0.2 to 10 wt.-%, most preferably in the range of 1 to 5 wt.-%, based on the total weight of the composition.

The amount of erythrulose (based on the active) in the compositions according to the present invention is preferably selected in the range of 0.1 to 5 wt.-%, more preferably in the range of 0.5 to 4 wt.-%, most preferably in the range of 1 to 3 wt.-%, based on the total weight of the composition.

The amount of dihydroxyacetone in the compositions according to the present invention is preferably selected in the range of 0.1 to 15 wt.-%, more preferably in the range of 0.5 to 12 wt.-%, most preferably in the range of 1 to 10 wt.-%, based on the total weight of the composition.

If erythrulose is used in combination with DHA, the cosmetic or dermatological composition preferably comprises about 1 to 5 wt.-% erythrulose and about 1 to 15 wt.-% DHA, such as advantageously about 1.5 wt.-% erythrulose and 3.5 wt.-% of DHA based on the total weight of the composition.

All percentages and ratios mentioned in this specification are by weight if nothing else is stated or evident.

Preferably, in all embodiments according to the present invention, the ratio (w/w) of the at least one self-tanning agent to the polysiloxane-based UV filter having a chromophore residue of the benzalmalonate type is advantageously selected in the range of 0.1:1 to 1:0.1, preferably in the range of 0.2:1 to 1:0.2, more preferably in the range of 0.5:1 to 1:0.5, most preferably in the range of about 1:1.

Thus, the invention also relates to compositions comprising a polysiloxane-based UV filter having a chromophore residue of the benzalmalonate type such as preferably polysilicone-15 in combination with erythrulose and/ or DHA as self-tanning agent, wherein the ratio (w/w) of the self-tanning agent to polysilicone-15 is selected in the range of 0.2:1 to 1:0.2, preferably in the range of 0.5:1 to 1 :0.5, such as in particular in the range of about 1:1.

The cosmetic or dermatological compositions according to the present invention can be prepared according to the state in the art. The compositions according to the invention additionally comprise a cosmetically or dermatologically acceptable carrier, vehicle or diluents.

The term cosmetically or dermatologically acceptable carrier refers to all carriers and/or excipients and/ or diluents conventionally used in cosmetic compositions.

The topical compositions according to the present invention are generally prepared by admixing the self-tanning agent and the polysiloxane-based UV filter having a chromophore residue of the benzalmalonate type in the amounts indicated herein with a suitable carrier.

Preferred are cosmetic or dermatological compositions for artificial/sunless tanning and/or browning of human skin. Preferably, the compositions which impart a self tanning effect to the skin are compositions for artificial/sunless tanning and/or browning, sunscreen compositions or moisturizers.

The self-tanning compositions in accordance with the invention can be provided in the form of creams, milks, gels, cream gels, oil-in-water emulsions, vesicular dispersions, fluid lotions, in particular vaporizable fluid lotions, or any other form generally used in cosmetics, in particular those usually suitable for self-tanning cosmetic compositions.

The compositions in accordance with the present invention can additionally comprise conventional cosmetic additives and adjuvants selected, in particular, from among fatty substances, organic solvents, ionic or nonionic thickeners, softeners, antioxidants, agents for combating free radicals, opacifiers, stabilizers, emollients, silicones, .alpha.-hydroxy acids, antifoaming agents, moisturizing agents, vitamins, insect repellents, substance P antagonists, anti-inflammatories, fragrances, preservatives, surfactants, fillers, polymers, propellants, basifying or acidifying agents, colorants or any other ingredient commonly used in the cosmetic and/or dermatological field, in particular for the production of self-tanning compositions in the form of emulsions.

The fatty substances can be an oil or a wax, or mixture thereof. By the term "oil" is intended a compound which is liquid at ambient temperature. By the term "wax" is intended a compound which is solid or substantially solid at ambient temperature and for which the melting point is generally greater than 35 °C.

Exemplary oils are mineral oils (liquid paraffin); vegetable oils (sweet almond, macadamia, blackcurrant seed or jojoba oil); synthetic oils, such as perhydrosqualene, fatty alcohols, acids or esters (such as the C.sub.12 -C.sub.15 alkyl benzoate marketed under the trademark "Finsolv TN" by Finetex, octyl palmitate, isopropyl lanolate or triglycerides, including those of capric/caprylic acids), or oxyethylenated or oxypropylenated fatty esters and ethers; silicone oils (cyclomethicone, polydimethylsiloxanes or PDMS); fluorinated oils; polyalkylenes and their mixtures.

Exemplary waxy compounds are paraffin wax, carnauba wax, beeswax or hydrogenated castor oil.

And exemplary organic solvents include the lower alcohols and polyols having at most 8 carbon atoms.

The thickeners are advantageously selected from among the crosslinked polyacrylic acids or modified or unmodified guar gums and celluloses, such as hydroxypropylated guar gum, methylhydroxyethylcellulose and hydroxypropylmethylcellulose.

The compositions according to this invention can additionally comprise further organic or inorganic light screening agents which are active in the UV-A and/or UV-B regions (absorbers), such light screening agents being water-soluble, fat-soluble or insoluble in commonly used cosmetic solvents.

Examples of UV-B or broadspectrum screening agents, i.e. substances having absorption maximums between about 290 nm and 340 nm may be organic or inorganic compounds. Organic UV-B or broadband screening agents are e.g. acrylates such as 2-ethylhexyl 2-cyano-3,3-diphenylacrylate (octocrylene, PARSOL^{®} 340), ethyl 2-cyano-3,3-diphenylacrylate and the like; camphor derivatives such as 4-methyl benzylidene camphor (PARSOL^{®} 5000), 3-benzylidene camphor, camphor benzalkonium methosulfate, polyacrylamidomethyl benzylidene camphor, sulfo benzylidene camphor, sulphomethyl benzylidene camphor, therephthalidene dicamphor sulfonic acid and the like; Cinnamate derivatives such as ethylhexyl methoxycinnamate (PARSOL^{®} MCX), ethoxyethyl methoxycinnamate, diethanolamine methoxycinnamate (PARSOL^{®} Hydro), isoamyl methoxycinnamate and the like as well as cinnamic acid derivatives bond to siloxanes; p-aminobenzoic acid derivatives, such as p-aminobenzoic acid, 2-ethylhexyl p-dimethylaminobenzoate, N-oxypropylenated ethyl p-aminobenzoate, glyceryl p-aminobenzoate; benzophenones such as benzophenone-3, benzophenone-4, 2,2',4,4'-tetrahydroxy-benzophenone, 2,2'-dihydroxy-4,4'-dimethoxybenzophenone and the like; esters of benzalmalonic acid such as di-(2-ethylhexyl) 4-methoxybenzalmalonate; esters of 2-(4-ethoxy-anilinomethylene)propandioic acid such as 2-(4-ethoxy anilinomethylene) propandioic acid diethyl ester as described in the European Patent Publication EP 0895 776F; drometrizole trisiloxane (Mexoryl XL); imidazole derivatives such as e.g. 2-phenyl benzimidazole sulfonic acid and its salts (PARSOL^{®}HS). Salts of 2-phenyl benzimidazole sulfonic acid are e.g. alkali salts such as sodium- or potassium salts, ammonium salts, morpholine salts, salts of primary, sec. and tert. amines like monoethanolamine salts, diethanolamine salts and the like; salicylate derivatives such as isopropylbenzyl salicylate, benzyl salicylate, butyl salicylate, ethylhexyl salicylate (PARSOL^{®} EHS, Neo Heliopan OS), isooctyl salicylate or homomenthyl salicylate (homosalate, PARSOL^{®} HMS, Neo Heliopan HMS) and the like; triazine derivatives such as ethylhexyl triazone (Uvinul T-150), diethylhexyl butamido triazone (Uvasorb HEB) and the like. Encapsulated UV-filters such as encapsulated ethylhexyl methoxycinnamate (Eusolex UV-pearls) or microcapsules loaded with UV-filters as e.g. dislosed in EP 1471995 and the like;

Examples of broad spectrum or UV A screening agents i.e. substances having absorption maximums between about 320 nm and 400 nm may be organic or inorganic compounds e.g. dibenzoylmethane derivatives such as 4-tert.-butyl-4'-methoxydibenzoyl-methane (PARSOL^{®} 1789), dimethoxydibenzoylmethane, isopropyldibenzoylmethane and the like; benzotriazole derivatives such as 2,2'-methylene-bis-(6-(2H-benzotriazole-2-yl)-4-(1,1,3,3,-tetramethylbutyl)-phenol (PARSOL^{®} MAX) and the like; bis-ethylhexyloxyphenol methoxyphenyl triazine (PARSOL^{®} SHIELD) and the like; phenylene-1,4-bis-benzimidazolsulfonic acids or salts such as 2,2-(1,4-phenylene)bis-(1H-benzimidazol-4,6-disulfonic acid) (Neoheliopan AP); amino substituted hydroxybenzophenones such as 2-(4-Diethylamino-2-hydroxy-benzoyl)-benzoic acid hexylester (Uvinul A plus) as described in the European Patent Publication EP 1046391; Ionic UV-A filters as described in the International Patent Publication WO2005080341 A1; Pigments such as microparticulated ZnO and the like. The term "microparticulated" refers to a particle size from about 5 nm to about 200 nm, particularly from about 15 nm to about 100 nm. The particles may also be coated by other metal oxides such as e.g. aluminum or zirconium oxides or by organic coatings such as e.g. polyols, methicone, aluminum stearate, alkyl silane. Such coatings are well known in the art.

As dibenzoylmethane derivatives have limited photostability it may be desirable to photostabilize these UV-A screening agents. Thus, the term "conventional UV-A screening agent" also refers to dibenzoylmethane derivatives such as e.g. PARSOL^{®} 1789 stabilized by, e.g. 3,3-Diphenylacrylate derivatives as described in the European Patent Publications EP 0 514 491 B1 and EP 0 780 119 A1; Benzylidene camphor derivatives as described in the US Patent No. 5,605,680; Organosiloxanes containing benzmalonate groups as described in the European Patent Publications EP 0358584 B1, EP 0538431 B1 and EP 0709080 A1.

The light screening agents are generally present in the compositions according to the invention in proportions ranging from 0.1 to 20 wt.-% with respect to the total weight of the composition and preferably ranging from 0.2 to 15 wt.-% with respect to the total weight.

Of course, one skilled in this art will take care to select the abovementioned optional additional compound or compounds and/or their amounts such that the advantageous properties intrinsically associated with the combination in accordance with the invention are not, or not substantially, detrimentally affected by the envisaged addition or additions.

The compositions according to the invention can be formulated according to techniques well known to this art, in particular those suited for the preparation of emulsions of oil-in-water or water-in-oil type.

These compositions can be provided in the form of a simple or complex emulsion (O/W, W/O, O/W/O or W/O/W), such as a cream or a milk, or in the form of a gel or of a cream gel, or in the form of a lotion, of a powder or of a solid tube and can optionally be packaged as an aerosol and provided in the form of a foam or spray.

The compositions according to the invention are preferably formulated as aqueous gels, an oil-in-water or water-in-oil emulsion.

In an advantageous embodiment, the compositions according to the present invention comprise from about 50% to about 99%, preferably from about 60% to about 98%, more preferably from about 70% to about 98%, such as in particular from about 80% to about 95% of a carrier, based on the total weight of the cosmetic composition.

In a particular advantageous embodiment, the carrier consists furthermore of at least 40 wt.-%, more preferably of at least 50 wt.-%, most preferably of at least 55 wt.-% of water, such as in particular of about 55 to about 90 wt.-% of water or even 75 to 90 wt.-% of water.

The cosmetic and/ or dermatological compositions according to the invention have a pH in the range of 3 to 10, preferably in the range of pH of 4 to 8, most preferred in the range of pH4to7.

The cosmetic and/ or dermatological compositions according to the present invention advantageously comprise further preservatives or preservative booster. Preferably, the additional preservatives respectively preservative booster is selected from the group consisting of phenoxyethanol, ethylhexylglycerin, hydroxyacetophenone, glyceryl caprylate, caprylyl glycol, 1,2-hexanediol, propanediol, propylene glycol as well as mixtures thereof.

Preferably, a preservative/ preservative booster is present in the compositions according to the present invention. Most preferably said preservatives/ preservative booster is phenoxyethanol and/ or ethylhexylglycerin, most preferably a mixture thereof.

When present, the preservative respectively preservative booster is preferably used in an amount of 0.01 to 2 wt.-%, more preferably in an amount of 0.05 to 1.5 wt.-%, most preferably in an amount of 0.1 to 1.25 wt.-%, or even 0.5 to 1 wt.-%, based on the total weight of the composition. It is particularly preferred, that the cosmetic compositions according to the invention does not contain any further/ other preservatives such as e.g. parabens and/ or methylisothiazolidine.

The invention also relates to a method for artificially tanning and/or browning the skin, which comprises topically applying an effective amount of a cosmetic composition as described above on the skin for such a period of time as is required to elicit the desired artificial/sunless tanning effect.

In a preferred embodiment, the invention relates to cosmetic or dermatological compositions comprising additionally a hydrocolloid selected from cellulose gum, xanthan gum, PVM/MA decadiene crosspolymer or hydroxyethyl cellulose.

The cosmetic and/ or dermatological compositions according to the invention are preferably applied at least once per day but can also be applied several times a day e.g. two or three times a day.

The amount of the cosmetic and/or dermatological composition which is to be applied to the skin depends on the concentration of the active ingredients in the compositions and the desired cosmetic or pharmaceutical effect. For example, application can be such that a crème is applied to the skin. A cream is usually applied in an amount of about 1 to 2 mg crème/cm² skin. The amount of the composition which is applied to the skin is, however, not critical, and if with a certain amount of applied composition the desired effect cannot be achieved, a higher concentration of the active ingredients can be used e.g. by applying more of the composition or by applying compositions which contain more active ingredient.

In order to further illustrate the present invention and the advantages thereof, the following specific example is given, it being understood that same is intended only as illustrative and in nowise limitative.

### Example1: Measuring tanning reactivity on human skin

The following formulations have been prepared:
1. Containing 5% ERYTHRULOSE from DSM Nutritional products (~4% of active)
2. Containing 2% PARSOL^{®} SLX (INCI: polysilicone-15)
3. Containing 5% ERYTHRULOSE from DSM Nutritional products (~4% of active) and 2% PARSOL^{®} SLX

| **Formulation** | **1** | **2** | **3** |
|---|---|---|---|
| **INCI Name** | wt.-% | wt.-% | wt.-% |
| AQUA | Ad 100 | Ad 100 | Ad 100 |
| PROPYLENE GLYCOL | 3 | 3 | 3 |
| PHENOXYETHANOL; ETHYLHEXYLGLYCERIN | 1 | 1 | 1 |
| **ERYTHRULOSE; AQUA (~80% in water)** | 5 | 0 | 5 |
| DIMETHICONE | 5 | 5 | 5 |
| **POLYSILICONE-15** | **0** | **2** | **2** |
| POLYQUATERNIUM-37; TRIDECETH-6; PROPYLENE GLYCOL DICAPRYLATE/DICAPRATE | 1.5 | 1.5 | 1.5 |

Five different human subjects applied the test formulations on their inner forearm on a circular area with a diameter of 5cm. For one application 50ul of formulation that was evenly spread by moving the fingertip on the circular test area until the cream felt to be absorbed. Four test areas were defined on the forearms (2 of them each side), one untreated, one for 5% Erythrulose (~ 4% active), one for the combination 5% Erythrulose (~ 4% active) and 2% PARSOL^{®} SLX and one for 2% PARSOL^{®} SLX only.

24 hours later the skin tone was measured on the test areas using a Chromameter (Minolta). The measured parameter was the luminosity (L- value) which reflects the brightness of the skin (a lower L values illustrates a darker skin). Three consecutive measurements were taken on each test area and the average value calculated per test area. The results are presented in table 1 & 2.

**Table 1: Luminosity values L that measured with the Chromameter after 24 h**

| **#** | **Reference 1 Untreated skin** | **Reference 2 Formulation 1** | **Reference 3 Formulation 2** | **Invention Formulation 3** |
|---|---|---|---|---|
| Subject 1 | 63.66 | 61.44 | 62.64 | 58.52 |
| Subject 2 | 68.34 | 66.56 | 67.61 | 65.58 |
| Subject 3 | 62.91 | 63.91 | 64.81 | 62.65 |
| Subject 4 | 60.90 | 62.21 | 60.48 | 60.36 |
| Subject 5 | 65.22 | 64.32 | 64.07 | 63.70 |
| Average | 64.21 | 63.69 | 63.92 | 62.16 |
| Difference to untreated | - | -0.52 | -0.28 | -2.04 |

Surprisingly the application of the combination of 5% Erythrulose and 2% PARSOL^{®} SLX led to a significantly improved (i.e. darker) skin tan compared to the effect resulting from Erythrulose alone.

### Example2: Transepidermal water loss (TEWL) on human skin

The same test setup as in example 1 was used for these measurements.

TEWL is the steady-state flux of liquid water diffusing though the stratum corneum. The TEWL is a measure for the stability and intactness of the skin barrier. A reduced TEWL value indicates an improved skin barrier leading to an improved skin moisturization.

The TEWL measurement was performed 24hours after the application using the Aquaflux Biox Systems Ltd device according to the manufacturer's instructions.

**Table 3: TEWL values (mg/m²h) after 24 h**

| **#** | **Reference 1 Untreated skin** | **Reference 2 Formulation 1** | **Reference 3 Formulation 3** | **Invention Formulation 2** |
|---|---|---|---|---|
| Subject 1 | 8.88 | 10.80 | 10.40 | 10.48 |
| Subject 2 | 9.14 | 11.24 | 9.93 | 11.34 |
| Subject 3 | 5.85 | 7.97 | 7.45 | 7.03 |
| Subject 4 | 11.25 | 12.06 | 12.03 | 10.23 |
| Subject 5 | 7.02 | 8.21 | 8.56 | 7.41 |
| Average of all | 8.43 | 10.06 | 9.67 | 9.30 |
| Difference to untreated | - | 1.63 | 1.25 | 0.87 |

Surprisingly the combination of 5% (∼4 % of active) Erythrulose and 2% PARSOL^{®} SLX showed a significant lower TEWL value compared to the use of Erythrulose respectively PARSOL^{®} SLX alone which illustrates the restoration of the barrier function of the skin compared to the treatment with the individual ingredients.

## Claims

1. A cosmetic or dermatological composition comprising erythrulose and a polysiloxane-based UV filter having a chromophore residue of the benzalmalonate type, **characterized in that** the polysiloxane based UV-filter having a chromophore residue of the benzalmalonate type is used as a distinct, separate UV-filter and not in the form of a coating on a titanium dioxide, which coating is adhered to the surface to the titanium dioxide and is polysilicone-15.

2. The cosmetic or dermatological composition according to claim 1, wherein the amount of the polysiloxane-based UV filter is selected in the range 0.1 to 15 wt.-%, preferably in the range of 0.5 to 10 wt.-%, most preferably in the range of 1 to 5 wt.-%, based on the total weight of the composition.

3. The cosmetic or dermatological composition according to any one of claims 1 to 2, wherein the amount of erythrulose is selected in the range of 0.1 to 20 wt.-%, preferably in the range of 0.2 to 10 wt.-%, most preferably in the range of 1 to 5 wt.-%, based on the total weight of the composition.

4. The cosmetic or dermatological composition according to any one of claims 1 to 3 further comprising at least one further light screening agent.

5. Non-therapeutic use of a composition as in any one of claims 1 to 4 for artificial/sunless tanning, browning of human skin and/or restoring and/ or improving the skin barrier function.

6. Non-therapeutic use according to claim 5, wherein the restoring and/ or improving of the skin barrier function comprises at least one of restoring, maintaining or reducing the skin dehydration rate and restoring, maintaining or promoting the skin hydration value.

7. Non-therapeutic use of a composition as in any one of claims 1 to 4 for the enhancement of the natural glow of the skin.

8. The cosmetic or dermatological composition as in any one of claims 1 to 4 for the use in the protection of human skin against UV-radiation.

9. Non-therapeutic use of a polysiloxane-based UV filter having a chromophore residue of the benzalmalonate type as defined in claim 1 for the use in restoring and/ or improving the barrier function of skin treated with erythrulose such as in particular to restore respectively to reduce the skin dehydration rate and to restore respectively improve the skin hydration value, compared to the treatment with the self-tanning agent alone.

## Patentansprüche

1. Kosmetische oder dermatologische Zusammensetzung umfassend Erythrulose und einen UV-Filter auf Polysiloxanbasis mit einem chromophoren Rest von dem Benzalmalonattyp, **dadurch gekennzeichnet, dass** der UV-Filter auf Polysiloxanbasis mit einem chromophoren Rest von dem Benzalmalonattyp als ein eigener, getrennter UV-Filter und nicht in der Form einer Beschichtung auf einem Titandioxid, welche Beschichtung an der Oberfläche des Titandioxids haftet, verwendet wird und Polysilicon-15 ist.

2. Kosmetische oder dermatologische Zusammensetzung gemäß Anspruch 1, wobei die Menge an dem UV-Filter auf Polysiloxanbasis ausgewählt ist aus dem Bereich von 0,1 bis 15 Gew.-%, vorzugsweise aus dem Bereich von 0,5 bis 10 Gew.-%, höchst bevorzugt aus dem Bereich von 1 bis 5 Gew.-%, bezogen auf das Gesamtgewicht der Zusammensetzung.

3. Kosmetische oder dermatologische Zusammensetzung gemäß einem der Ansprüche 1 bis 2, wobei die Menge an Erythrulose ausgewählt ist aus dem Bereich von 0,1 bis 20 Gew.-%, vorzugsweise aus dem Bereich von 0,2 bis 10 Gew.-%, höchst bevorzugt aus dem Bereich von 1 bis 5 Gew.-%, bezogen auf das Gesamtgewicht der Zusammensetzung.

4. Kosmetische oder dermatologische Zusammensetzung gemäß einem der Ansprüche 1 bis 3, ferner umfassend wenigstens ein weiteres Lichtschutzmittel.

5. Nichttherapueutische Verwendung einer Zusammensetzung gemäß einem der Ansprüche 1 bis 4 zur künstlichen/sonnenlösen Bräunung, Bräunung von menschlicher Haut und/oder Wiederherstellung und/oder Verbesserung der Hautbarrierefunktion.

6. Nichttherapueutische Verwendung gemäß Anspruch 5, wobei die Wiederherstellung und/oder Verbesserung der Hautbarrierefunktion wenigstens eines von Wiederherstellen, Bewahren oder Verringern der Hautdehydratationsrate und Wiederherstellen, Bewahren oder Verringern des Hauthydratationswerts umfasst.

7. Nichttherapueutische Verwendung gemäß einem der Ansprüche 1 bis 4 zur Verbesserung das natürlichen Strahlens der Haut.

8. Kosmetische oder dermatologische Zusammensetzung gemäß einem der Ansprüche 1 bis 4 zur Verwendung zum Schutz von menschlicher Haut gegen UV-Strahlung.

9. Nichttherapeutische Verwendung eines UV-Filters auf Polysiloxanbasis mit einem chromophoren Rest von dem Benzalmalonattyp gemäß Anspruch 1 zur Verwendung zur Wiederherstellung und/oder Verbesserung der Barrierefunktion von mit Erythrulose behandelter Haut, wie z.B. insbesondere zur Wiederherstellung bzw. Verringerung der Hautdehydratationssrate und zur Wiederherstellung bzw. Verbesserung des Hauthydratationswerts im Vergleich zu der Behandlung mit dem Selbstbräunungsmittel allein.

## Revendications

1. Composition cosmétique ou dermatologique comprenant de l'érythrulose et un filtre UV à base de polysiloxane ayant un radical chromophore du type benzalmalonate, **caractérisée en ce que** le filtre UV à base de polysiloxane ayant un radical chromophore du type benzalmalonate est utilisé comme un filtre UV distinct, séparé et non sous la forme d'un revêtement sur un dioxyde de titane, lequel revêtement est collé à la surface au dioxyde de titane et est une polysilicone-15.

2. Composition cosmétique ou dermatologique selon la revendication 1, la quantité du filtre UV à base de polysiloxane étant choisie dans la plage 0,1 à 15 % en poids, préférablement dans la plage de 0,5 à 10 % en poids, le plus préférablement dans la plage de 1 à 5 % en poids, sur la base du poids total de la composition.

3. Composition cosmétique ou dermatologique selon l'une quelconque des revendications 1 à 2, la quantité d'érythrulose étant choisie dans la plage de 0,1 à 20 % en poids, préférablement dans la plage de 0,2 à 10 % en poids, le plus préférablement dans la plage de 1 à 5 % en poids, sur la base du poids total de la composition.

4. Composition cosmétique ou dermatologique selon l'une quelconque des revendications 1 à 3 comprenant en outre au moins un autre agent écran de la lumière.

5. Utilisation non thérapeutique d'une composition comme dans l'une quelconque des revendications 1 à 4 pour un bronzage artificiel/sans soleil, le brunissement de la peau humaine et/ou la restauration et/ou l'amélioration de la fonction barrière de la peau.

6. Utilisation non thérapeutique selon la revendication 5, la restauration et/ou l'amélioration de la fonction barrière de la peau comprenant au moins l'un parmi la restauration, le maintien ou la réduction du taux de déshydratation de la peau et la restauration, le maintien ou la favorisation de la valeur d'hydratation de la peau.

7. Utilisation non thérapeutique d'une composition comme dans l'une quelconque des revendications 1 à 4 pour l'augmentation de la brillance naturelle de la peau.

8. Composition cosmétique ou dermatologique comme dans l'une quelconque des revendications 1 à 4 pour l'utilisation dans la protection de la peau humaine contre un rayonnement UV.

9. Utilisation non thérapeutique d'un filtre UV à base de polysiloxane ayant un radical chromophore du type benzalmalonate tel que défini dans la revendication 1 pour l'utilisation dans la restauration et/ou l'amélioration de la fonction barrière de la peau traitée par de l'érythrulose tel qu'en particulier pour restaurer, respectivement pour réduire, le taux de déshydratation de la peau et pour restaurer, respectivement améliorer, la valeur d'hydratation de la peau, par comparaison avec le traitement avec l'agent auto-bronzant seul.
